# EUROPEAN PATENT APPLICATION

(11) **EP 2 868 273 A1**
(43) Date of publication of application: **06.05.2015**
(21) Application number: 14191776.5
(22) Date of filing: 04.11.2014
(51) Int. Cl.: A61B 5/11, G01C 22/00, G06Q 10/10

(54) **Activity detection and analytics**

(30) Priority: 04.11.2013 US 201361899794 P; 06.01.2014 US 201414148571
(71) Applicant: InvenSense, Inc., San Jose, CA 95110 (US)
(72) Inventor: Gangumalla, Vamshi R., San Jose, CA 95110 (US); Katingari, Karthik, San Jose, CA 95110 (US)
(74) Representative: Smith, Jeremy Robert

(57) **Abstract**

A method and system for activity detection and analytics are disclosed. The method comprises determining a context and providing the determined context and one or more outputs from at least one sensor to an analytics engine to provide analytics results. The system includes at least one sensor and a processing system coupled to the at least one sensor, wherein the processing system includes an analytics engine that is configured to receive a determined context and one or more outputs from at least one sensor to provide analytics results.

## Description

### CROSS-REFERENCE TO RELATED APPLICATION

This application claims benefit under 35 USC 119(e) of U.S. Provisional Patent Application No. 61/899,794, filed on November 4, 2013, entitled "METHOD TO IMPROVE ACTIVITY DETECTION AND ANALYTICS," which is incorporated herein by reference in its entirety.

### FIELD OF THE INVENTION

The present invention relates to sensor devices, and more particularly, to sensor devices utilized for activity detection and analytics.

### BACKGROUND

Sensors, sensor devices, and wearable devices are utilized in a variety of applications including the detection and identification of user's activities (e.g. walking, running). Conventional sensor devices and activity classification devices suffer from various inaccuracies including false positive detection of the user's activities. In certain situations such as being in a vehicle or bike riding, conventional pedometers suffer from over-counting during activities where the user is not taking steps because repetitive acceleration data signatures are mistaken for steps. Therefore, there is a strong need for a solution that overcomes the aforementioned issues. The present invention addresses such a need.

### SUMMARY OF THE INVENTION

A method and system for activity detection and analytics are disclosed. In a first aspect, the method comprises determining a context and providing the determined context and one or more outputs from at least one sensor to an analytics engine to provide analytics results.

In a second aspect, the system includes at least one sensor and a processing system coupled to the at least one sensor, wherein the processing system includes an analytics engine that is configured to receive a determined context and one or more outputs from at least one sensor to provide analytics results.

### BRIEF DESCRIPTION OF THE DRAWINGS

The accompanying figures illustrate several embodiments of the invention and, together with the description, serve to explain the principles of the invention. One of ordinary skill in the art readily recognizes that the embodiments illustrated in the figures are merely exemplary, and are not intended to limit the scope of the present invention.
Figure 1 illustrates a system that includes a Motion Processing Unit (MPU) in accordance with an embodiment.
Figure 2 illustrates a method for analytics by a wearable device in accordance with an embodiment.
Figure 3 illustrates a wearable device for activity analytics in accordance with an embodiment.
Figure 4 illustrates a wearable device for pedometer analytics in accordance with an embodiment.
Figure 5 illustrates a wearable device for activity analytics in accordance with an embodiment.
Figure 6 illustrates a wearable device for activity analytics in accordance with an embodiment.
Figure 7 illustrates example wearable devices and remote devices arranged in relation to an integrated system of the present invention, in accordance with one or more embodiments.

### DETAILED DESCRIPTION

The present invention relates to sensor devices, and more particularly, to sensor devices utilized for activity detection and analytics. The following description is presented to enable one of ordinary skill in the art to make and use the invention and is provided in the context of a patent application and its requirements. Various modifications to the preferred embodiment and the generic principles and features described herein will be readily apparent to those skilled in the art. Thus, the present invention is not intended to be limited to the embodiments shown but is to be accorded the widest scope consistent with the principles and features described herein.

A method and system in accordance with the present invention provide for a wearable device and platform that detects and classifies a user's activity based upon determined contexts and an algorithm to guard against erroneous activity classifications and to provide for accurate activity classifications when present. By integrating sensors, an activity recognition engine that includes the algorithm, and an analytics engine into the wearable device, activities are detected with a certain confidence to adaptively change decision parameters for the analytics engine that estimates the analytics.

In the described embodiments, "raw data" refers to measurement outputs from sensors which are not yet processed. "Motion data" refers to processed sensor data. Processing of the data by the wearable device may be accomplished by applying a sensor fusion algorithm or applying any other algorithm. In the case of the sensor fusion algorithm, data from one or more sensors are combined to provide an orientation of the device including but not limited to heading angle and/or confidence value. The predefined reference in world coordinates refers to a coordinate system where one axis of the coordinate system aligns with the earth's gravity, a second axis of the coordinate system coordinate points towards magnetic north, and the third coordinate is orthogonal to the first and second coordinates.

To describe the features of the present invention in more detail, refer now to the following description in conjunction with the accompanying Figures.

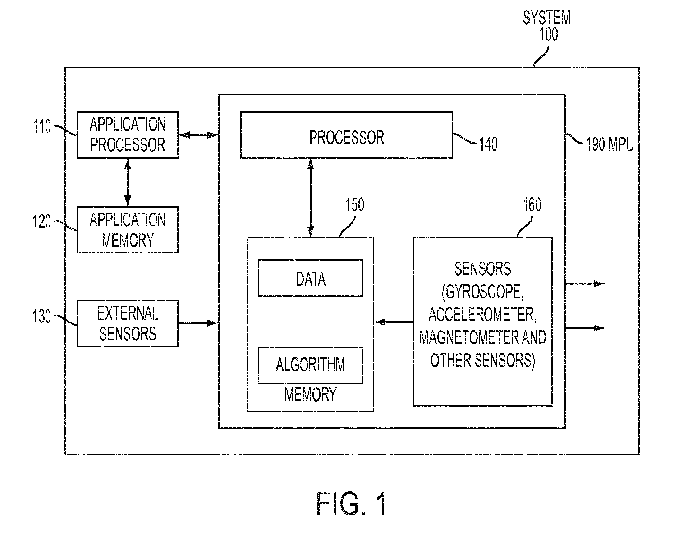

In one embodiment, an integrated system of the present invention includes a motion tracking device (also referred to as a Motion Processing Unit (MPU)) that includes sensors and electronic circuits. FIG. 1 illustrates a system 100 that includes a Motion Processing Unit (MPU) 190 in accordance with an embodiment. The system 100 includes the MPU 190, an application processor 110, an application memory 120, and external sensors 130. In one embodiment, the MPU 190 includes a processor 140, a memory 150, and sensors 160. In another embodiment, the MPU 190 includes control logic and other structures.

In FIG. 1, the memory 150 is shown to store instructions to execute algorithms, raw data, and/or processed sensor data received from the sensors 160 and/or the external sensors 130. The algorithms may include sensor fusion algorithm, activity detection algorithm, analytics, or similar algorithms. In one embodiment, the sensors 160 and/or the external sensors 130 include any of accelerometer, gyroscope, magnetometer, solid-state sensor, pressure sensor, microphone, proximity sensor, haptic sensor, ambient light sensor, and other sensors.

In one embodiment, the sensors 160 and external sensors 130 provide a measurement along three axes that are orthogonal relative to each other, referred to as a 9-axis device. In other embodiments, the sensors 160 and/or external sensors 130 may not provide measurements along one or more axis. In one embodiment, the electronic circuits receive and processor measured outputs (e.g. sensor data) from one or more sensors. In another embodiment, the sensor data is processed on a processor on a different substrate/chip.

In one embodiment, the sensors 160 are formed on a first substrate (e.g. a first silicon substrate) and the electronic circuits are formed on a second substrate (e.g. a second silicon substrate). In one embodiment, the first substrate is vertically stacked, attached and electrically connected to the second substrate in a single semiconductor chip. In another embodiment, the first substrate is vertically stacked, attached and electrically connected to the second substrate in a single package. In yet another embodiment, the first and second substrates are electrically connected and placed next to each other in a single package.

In some embodiments, the processor 140, the memory 150, and the sensors 160 are formed on different chips and in other embodiments, the processor 140, the memory 150, and the sensors 160 are formed and reside on the same chip. In yet other embodiments, a sensor fusion algorithm, activity detection algorithm and analytics that is employed in calculating the orientation, activity detection algorithm and analytics are performed externally to the processor 140 and the MPU 190. In still other embodiments, the sensor fusion is determined by the MPU 190. In yet another embodiment, sensor fusion, activity detection algorithm, and analytics are determined both by the processor 140 and the application processor 110.

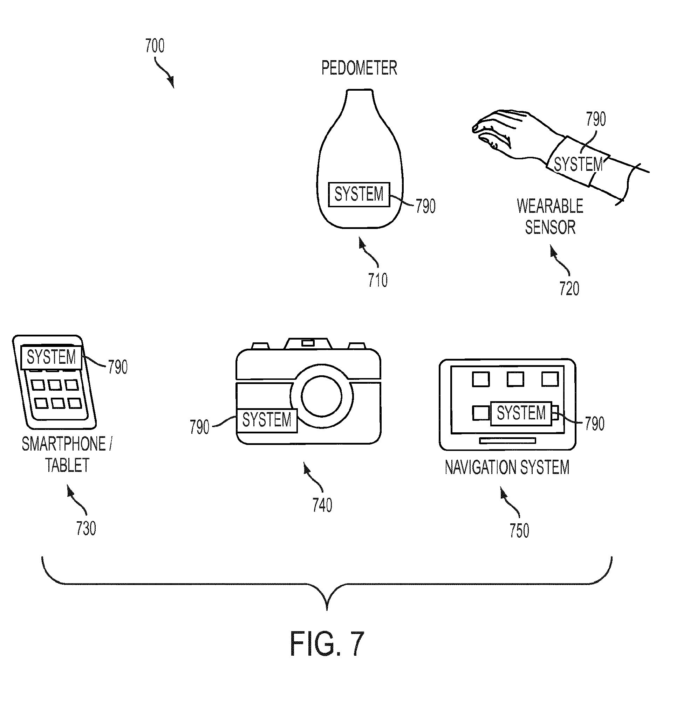

In one embodiment, the processor 140 executes code, according to an algorithm in the memory 150, to process data that is stored in the memory 150 and that is detected by the sensors 160. In another embodiment, the application processor 110 sends to or retrieves from the application memory 120 and is coupled to the processor 140. In one embodiment, the application in the processor 140 can be one of a variety of application types including but not limited to a navigation system, compass accuracy application, remote control application, 3-dimensional camera application, industrial automation application, and any other motion tracking type application. For the 3-dimensional camera application, a bias error or sensitivity error is estimate by the processor 140. It is understood that this is not an exhaustive list of applications and that other applications are contemplated. It will be appreciated that these and other embodiments of the present invention are readily understood as a result of the present invention wherein the system 100 of FIG. 1 may be incorporated into the described exemplars of FIG. 7.

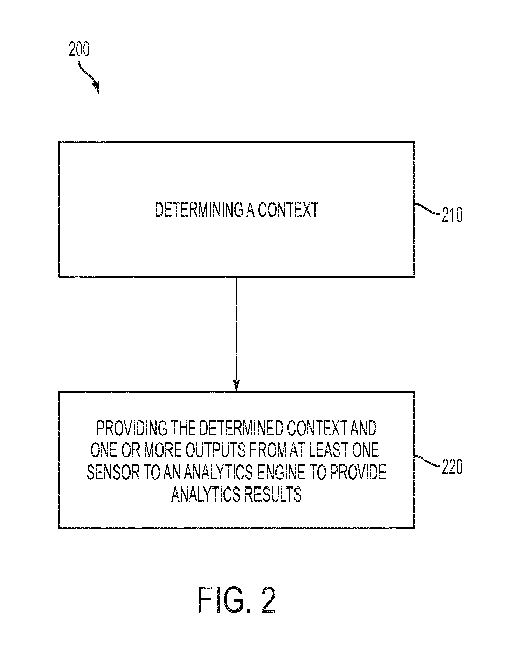

FIG. 2 illustrates a method 200 for analytics by a wearable device in accordance with an embodiment. The method 200 includes determining a context via step 210 and providing the determined context and one or more outputs from at least one sensor to an analytics engine to provide analytics results via step 220. In one embodiment, the context is determined by an activity recognition engine that receives one or more outputs from at least one sensor. In one embodiment, the analytics engine is comprised of any of at least one software component and at least one hardware component.

In one embodiment, the determined context is utilized to classify an activity of the user to provide further analytics results. Examples of activities and analytics are listed below.

| Activity/Context | Analytics |
|---|---|
| Ball sports, soccer, basketball, football, Tennis, Golf, Squash, Bowling, basing etc. | (Spin, Angle change, trajectory, Swing, jump, trajectory, pitching type |
| Number of Jumps | Any sports |
| Running/Walking | (acceleration, speed, distance, angular acceleration, velocity, stair steps |
| Gym | Crunches, Lifts, Curls, balance, level, curving |
| Swimming | Number of stocks per minute, angle of incidence/follow-through of the stock |
| Boating | Rowing, paddling |
| Skiing | Speed, Jumps, |
| Figure Skating | Speed Jumps, Spins, Lifts, Turns |

In one embodiment, the analytics engine and the activity recognition engine receive the one or more outputs from the same sensor. In another embodiment, the analytics engine and the activity recognition engine receive the one or more outputs from different sensors.

In one embodiment, the analytics engine utilizes a threshold to provide the analytics results. In one embodiment, the threshold is a predetermined and preset value that is based upon previous machine learning data sets and/or other sampling techniques. In another embodiment, the threshold is dynamically and continuously adjusted based upon the classified activity that is outputted from the activity recognition engine and that serves as an input into the analytics engine.

In one embodiment, the classified activity that is determined by the activity recognition engine includes a variety of activities including but not limited to biking, running, walking, driving, and sleeping. In one embodiment, the analytic results that are determined by the analytics engine includes a variety of results including but not limited to steps per minute (SPM), number of steps, distance, speed, stride length, energy, calories, heart rate, and exercise counts. In one embodiment, the analytics results that are outputted by the analytics engine are utilized by the activity recognition engine to determine the classified activity. In another embodiment, the classified activity is determined in accordance with a context that has been established by the wearable device, wherein the context includes a variety of contexts but not limited to particular situations, environments, and controlling activities of the user such as gesturing.

In one embodiment, the gesturing of the user that establishes the context includes but is not limited to any of a touch, button, tap, signature, audio, command operation, image, bio signal, heart rate monitor, and movement. In this embodiment, the wearable device includes a gesture detector that is utilized to detect contact of a user with a touch sensor of the wearable device. The gesture detector may also comprise an accelerometer to detect acceleration data of the system/user and a gyroscope to detect rotation of the system/user, wherein the accelerometer and the gyroscope are utilized in combination to generate motion data.

In one embodiment, the activity recognition engine and the analytics engine are capable of receiving user input and instructions. In one embodiment, the user input and instructions include but are not limited to threshold values, activity classification categories, and time periods for analysis.

In one embodiment, the wearable device includes at least one sensor and a processing system (e.g. processor) coupled to the at least one sensor. In one embodiment, the processing system includes an activity recognition engine and an analytics engine. In another embodiment, the wearable device further includes a power management unit that is controlled by any of the activity recognition engine, the determined context, the classified activity, the analytics engine, and the analytics results. In one embodiment, the at least one sensor is dynamically selected based on any of the activity recognition engine, the determined context, the classified activity, the analytics engine, and the analytics results.

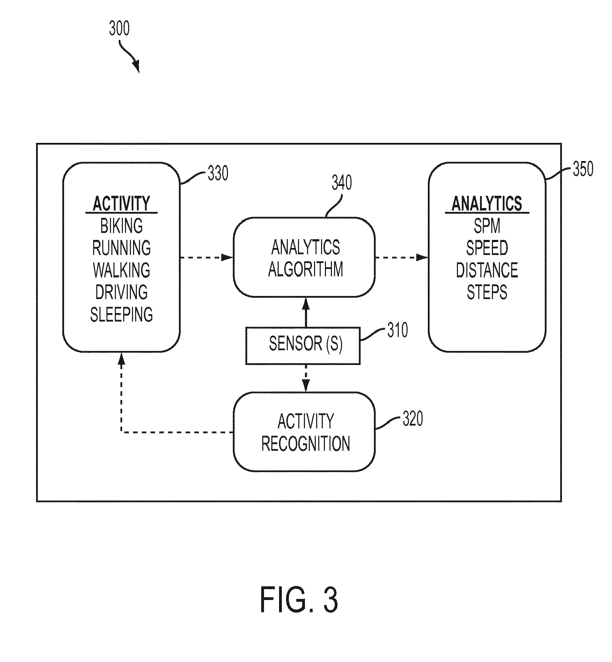

FIG. 3 illustrates a wearable device 300 for activity analytics in accordance with an embodiment. In FIG. 3, the wearable device 300 includes sensors 310, an activity recognition module 320, a classified activity 330, an analytics algorithm module 340, and a plurality of analytics 350 outputs. The wearable device 300 is attached to a user to detect various data and signals via the sensors 310 including but not limited to accelerometer, gyroscope, magnetometer, pressure, GPS, and heart rate data. The detected data is transmitted to the activity recognition module 320 that utilizes activity recognition (AR) algorithms to detect and to classify the user's activity and context into the classified activity 330.

The classified activity 330 includes but is not limited to activities such as biking, running, walking, driving, sleeping, and gestures. The classified activity 330 and data from the sensors 310 are both input into the analytics algorithm module 340 that determines a plurality of analytics 350 outputs including but not limited to steps per minute (SPM), number of steps, distance, speed, stride length, energy, calories, heart rate, and exercise counts. In another embodiment, only one of the classified activity 330 and data from the sensors 310 are input into the analytics algorithm module 340 for processing. In one embodiment, the activity recognition module 320 that detects the user's activity and the analytics algorithm module 340 that determines various analytics related to classified activity 330 can be executed on the same processor 140 or the same application processor 110 of the system 100 of FIG. 1 or on different processors.

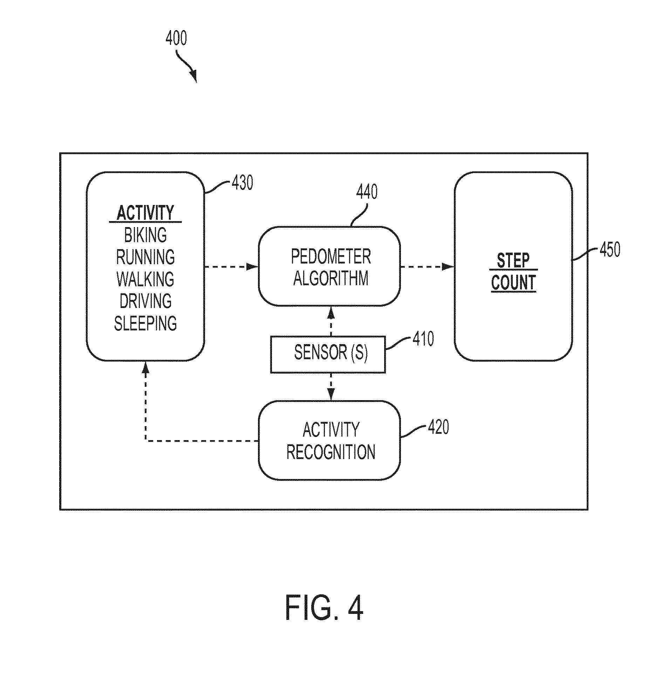

FIG. 4 illustrates a wearable device 400 for pedometer analytics in accordance with an embodiment. In FIG. 4, the wearable device 400 includes sensors 410, an activity recognition module 420, a classified activity 430, a pedometer algorithm module 440, and a step count 450 output. The wearable device 400 is attached to a user to detect various data and signals via the sensors 410 including but not limited to accelerometer, gyroscope, magnetometer, pressure, GPS, and heart rate data. The detected data is transmitted to the activity recognition module 420 that utilizes activity recognition (AR) algorithms to detect and to classify the user's activity and context into the classified activity 430.

The classified activity 430 includes but is not limited to activities such as biking, running, walking, driving, sleeping, and gestures. The classified activity 430 and data from the sensors 410 are both inputted into the pedometer algorithm module 440 that determines a step count 450 output. In another embodiment, only one of the classified activity 430 and data from the sensors 410 are inputted into the pedometer algorithm module 440 for processing. In one embodiment, the activity recognition module 420 that detects the user's activity and the pedometer algorithm module 440 that determines a step count related to the classified activity 430 can be executed on the same processor 140 or the same application processor 110 of the system 100 of FIG. 1 or on different processors.

In one embodiment, the activity/pedometer analytics modules 340/440 are dynamically and automatically adjusted based upon the received classified activity 330/430. For example, if the classified activity 330/430 is determined to be biking, the activity/pedometer analytics module 340/440 is adjusted to increase a peak threshold and increase the cadence count so that the activity/pedometer analytics module 340/440 is immune or less sensitive to various analytics such as counting steps. If the classified activity 330/440 is determined to be persistent walking, the activity/pedometer analytics module 340/440 is adjusted to decrease a peak threshold and decrease the cadence count so that the activity/pedometer analytics module 340/440 is more accurate in the determination of various analytics such as counting steps.

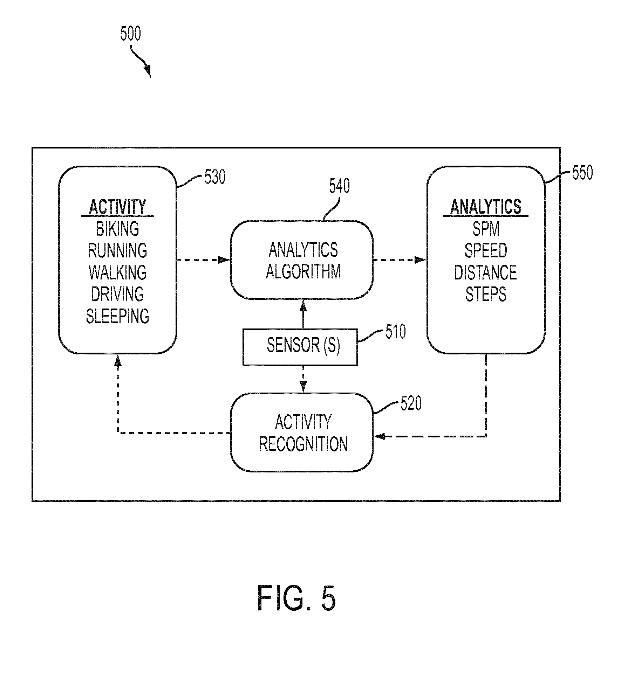

FIG. 5 illustrates a wearable device 500 for activity analytics in accordance with an embodiment. In FIG. 5, the wearable device 500 is similar to the diagram 300 of FIG. 3. In addition, in FIG. 5, the plurality of analytics 550 outputs that are determined by the analytics algorithm module 540 are inputted and fed back into the activity recognition module 520 to improve the activity detection confidential level. The activity recognition module 520 is constantly and automatically updated via a tight control loop mechanism to more accurately classify various activities and contexts of the user which in turn enables the analytics algorithm module 540 to more accurately determine various analytics such as steps per minute (SPM).

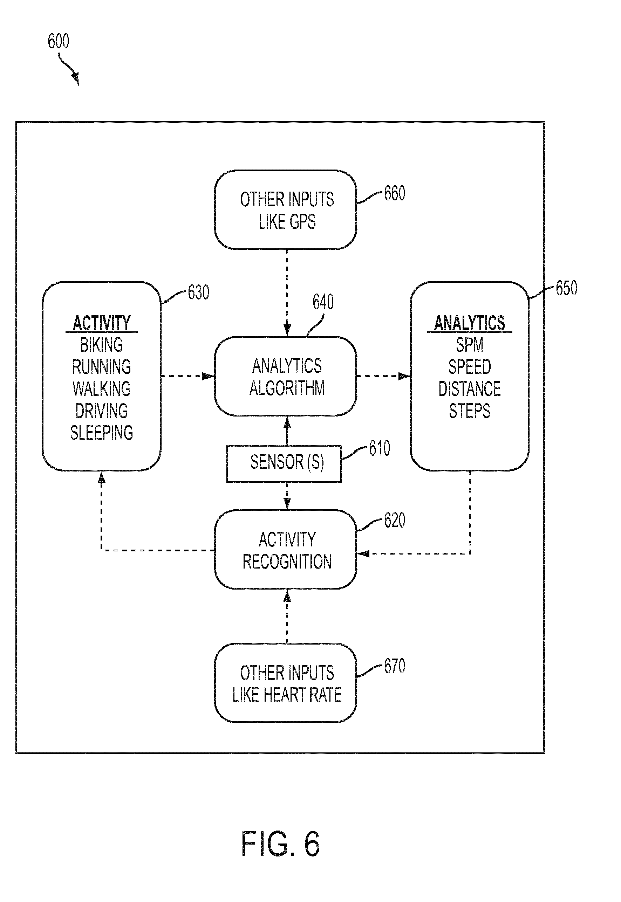

FIG. 6 illustrates a wearable device 600 for activity analytics in accordance with an embodiment. In FIG. 6, the wearable device 600 is similar to the diagram 500 of FIG. 5. In addition, in FIG. 6, the tight feedback loop mechanism is supplemented with other inputs 660 that are fed into the activity algorithm module 640 and with other inputs 670 that are fed into the activity recognition module 620. The other inputs 660 include but are not limited to GPS and the other inputs 670 include but are not limited to heart rate. The other inputs 660 and 670 are utilized by the wearable device 600 to continuously improve upon the accuracy of the activity recognition module 620 and the activity algorithm module 640. The classified activity 630 is utilized by the wearable device 600 to fine tune and update the algorithm that is utilized by the activity algorithm module 640 to determine the plurality of analytics 650 outputs.

In one embodiment, the classified activity and the plurality of analytics outputs are stored internally by the wearable device system in the memory. In another embodiment, the classified activity and the plurality of analytics outputs are transmitted by the wearable device to a difference device or a cloud computer system and network for storage and displaying on a screen. The different device or the cloud computer system and network utilize the compiled and stored data received from a wearable device to communicate with the wearable device and update and improve upon the activity recognition engine/module and the analytics algorithm engine/module.

FIG. 7 illustrates example wearable devices and remote devices 700 arranged in relation to an integrated system 790 of the present invention, in accordance with one or more embodiments. In FIG. 7, the example wearable devices include a pedometer device 710 with an integrated system 790, a wearable sensor 720 with the integrated system 790, a smartphone/tablet 730 with the integrated system 790, a camera 740 utilized in a remote device in communication with the integrated system 790, and a navigation system 750 with the integrated system 790. The wearable sensor 720 can be worn on a variety of user locations include the wrist. The navigation system 750 is capable of communication and is positioned as a smart media.

As above described, a method and system in accordance with the present invention utilizes a wearable platform and a Motion Processing Unit (MPU) to detect various data signals via sensors and to analyze the detected data signals for the automatic and continuous classification into various activities. By integrating various sensors, an activity recognition engine, and an analytics engine that includes an algorithm into the wearable device, various user activities are classified (e.g. walking, running, biking, etc.) and various metrics (e.g. step count, speed, distance, etc.) associated with that activity classification are determined. The MPU updates the algorithm utilized by the analytics engine based upon the outputted activity classifications and previous analytics metric determinations. It will be appreciated that the present invention has many implementations and uses not expressly stated herein.

A method and system for activity classification and analytics by a wearable device have been disclosed. Embodiments described herein can take the form of an entirely hardware implementation, an entirely software implementation, or an implementation containing both hardware and software elements. Embodiments may be implemented in software, which includes, but is not limited to, application software, firmware, resident software, microcode, etc. Embodiments described herein may also take the form where the entirety of the wearable device, sensors, and one or more remote devices or servers are co-located or integrated into the same or proximate device. In such an embodiment, the entirety of the present invention is integrated into one device. A method and system in accordance with the present invention is not so limited however.

The steps described herein may be implemented using any suitable controller or processor, and software application, which may be stored on any suitable storage location or computer-readable medium. The software application provides instructions that enable the processor to perform the functions described herein.

Furthermore, embodiments may take the form of a computer program product accessible from a computer-usable or computer-readable medium providing program code for use by or in connection with a computer or any instruction execution system. For the purposes of this description, a computer-usable or computer-readable medium can be any apparatus that can contain, store, communicate, propagate, or transport the program for use by or in connection with the instruction execution system, apparatus, or device.

The medium may be an electronic, magnetic, optical, electromagnetic, infrared, semiconductor system (or apparatus or device), or a propagation medium. Examples of a computer-readable medium include a semiconductor or solid state memory, magnetic tape, a removable computer diskette, a random access memory (RAM), a read-only memory (ROM), non-volatile read/write flash memory, a rigid magnetic disk, and an optical disk. Current examples of optical disks include DVD, compact disk-read-only memory (CD-ROM), and compact disk - read/write (CD-R/W).

Although the present invention has been described in accordance with the embodiments shown, one of ordinary skill in the art will readily recognize that there could be variations to the embodiments and those variations would be within the spirit and scope of the present invention. Accordingly, many modifications may be made by one of ordinary skill in the art without departing from the spirit and scope of the appended claims.

## Claims

1. A computer implemented method comprising:
determining a context; and
providing the determined context and one or more outputs from at least one sensor to an analytics engine to provide analytics results.

2. The computer implemented method of claim 1, wherein the context is determined by an activity recognition engine.

3. The computer implemented method of claim 2, wherein the activity recognition engine receives one or more outputs from at least one sensor.

4. The computer implemented method of claim 3, wherein the analytics engine and the activity recognition engine receive the one or more outputs from the same sensor.

5. The computer implemented method of claim 3, wherein the analytics engine and the activity recognition engine receive the one or more outputs from different sensors.

6. The computer implemented method of claim 2, further comprising:
classifying an activity by the activity recognition engine based on the determined context to provide further analytics results.

7. The computer implemented method of claim 6, wherein the classified activity includes any of biking, running, walking, driving, standing, sitting, and sleeping.

8. The computer implemented method of claim 6, wherein the analytics engine utilizes a change in threshold, frequency, and cut-off frequency to provide the analytics results.

9. The computer implemented method of claim 8, wherein the threshold is dynamically adjusted based upon the classified activity.

10. The computer implemented method of claim 6, wherein the analytics results are utilized by the activity recognition engine to determine the classified activity.

11. The computer implemented method of claim 1, wherein the at least one sensor is any of an accelerometer, gyroscope, pressure sensor, and other sensor.

12. The computer implemented method of claim 1, wherein the analytics engine is comprised of a software component and a hardware component.

13. The computer implemented method of claim 1, wherein the analytics results include any of steps per minute (SPM), number of steps, distance, speed, stride length, energy, calories, heart rate, and exercise counts.

14. The computer implemented method of claim 2, wherein any of the activity recognition engine and the analytics engine are capable of receiving user input and instructions.

15. The computer implemented method of claim 6, wherein a power management unit is controlled by any of the activity recognition engine, the determined context, the classified activity, the analytics engine, and the analytics results.

16. The computer implemented method of claim 6, wherein the at least one sensor is dynamically selected based on any of the activity recognition engine, the determined context, the classified activity, the analytics engine, and the analytics results.

17. A device comprising:
at least one sensor;
a processing system coupled to the at least one sensor, wherein the processing system includes an analytics engine that is configured to receive a determined context and one or more outputs from at least one sensor to provide analytics results.

18. The device of claim 17, wherein the processing system further comprises an activity recognition engine to determine the context and to provide the determined context to the analytics engine.

19. The device of claim 18, wherein the activity recognition engine receives one or more outputs from at least one sensor.

20. The device of claim 19, wherein the analytics engine and the activity recognition engine receive the one or more outputs from the same sensor.

21. The device of claim 19, wherein the analytics engine and the activity recognition engine receives the one or more outputs from different sensors.

22. The device of claim 18, wherein the activity recognition engine classifies an activity based on the determined context to provide further analytic results.

23. The device of claim 22, wherein the classified activity includes any of biking, running, walking, driving, standing, sitting, and sleeping.

24. The device of claim 17, wherein the analytics engine utilizes a change in threshold, frequency, and cut-off frequency to provide the analytics results.

25. The device of claim 24, wherein the threshold is dynamically adjusted based upon the classified activity.

26. The device of claim 22, wherein the analytics results are utilized by the activity recognition engine to determine the classified activity.

27. The device of claim 17, wherein the at least one sensor is any of an accelerometer, gyroscope, pressure sensor, and other sensor.

28. The device of claim 17, wherein the analytics engine is comprised of a software component and a hardware component.

29. The device of claim 17, wherein the analytics results include any of steps per minute (SPM), number of steps, distance, speed, stride length, energy, calories, heart rate, and exercise counts.

30. The device of claim 18, wherein any of the activity recognition engine and the analytics engine are capable of receiving user input and instructions.

31. The device of claim 22, further comprising a power management unit that is controlled by any of the activity recognition engine, the determined context, the classified activity, the analytics engine, and the analytics results.

32. The device of claim 22, wherein the at least one sensor is dynamically selected based on any of the activity recognition engine, the determined context, the classified activity, the analytics engine, and the analytics results.
